## Europäisches Patentamt
## European Patent Office
## Office européen des brevets

(11) Veröffentlichungsnummer: **0 222 248**
**B1**

(12) **EUROPÄISCHE PATENTSCHRIFT**

(45) Veröffentlichungstag der Patentschrift:
28.06.89

(51) Int. Cl.⁴: **C07C 15/44,** C07C 17/20,
C07C 7/04

(21) Anmeldenummer: 86114922.7

(22) Anmeldetag: 27.10.86

(54) Verfahren zur Destillation von styrol.

(30) Priorität: 09.11.85 DE 3539776

(43) Veröffentlichungstag der Anmeldung:
20.05.87 Patentblatt 87/21

(45) Bekanntmachung des Hinweises auf die Patenterteilung:
28.06.89 Patentblatt 89/26

(84) Benannte Vertragsstaaten:
DE FR IT NL

(56) Entgegenhaltungen:
EP-A- 0 062 626
EP-A- 0 160 553
DE-A- 3 203 418
DE-A- 3 247 813
DE-B- 1 040 020
GB-A- 971 976
US-A- 4 177 110

(73) Patentinhaber: BASF Aktiengesellschaft,
Carl-Bosch-Strasse 38, D-6700 Ludwigshafen(DE)

(72) Erfinder: Lausberg, Dietrich, Dr., Merziger Strasse 21,
D-6700 Ludwigshafen(DE)
Erfinder: Lieb, Manfred, Radestrasse 5,
D-6700 Ludwigshafen(DE)
Erfinder: Moeckel, Hubert, Fliesstrasse 39,
D-6701 Ellerstadt(DE)
Erfinder: Uhr, Hermann, Max-Beckmann-Strasse 22c,
D-6710 Frankenthal(DE)

**Beschreibung**

Die Erfindung betrifft ein Verfahren zur Destillation von Styrol unter Vermeidung unerwünschter Polymerisation in Destillationskolonnen mit Verstärkungsteil, wobei als Kolonnenzulauf ein Gemisch mit einem Gehalt an Styrol und Ethylbenzol in die Kolonne eingespeist wird und wobei ferner mindestens ein Polymerisationsinhibitor verwendet wird.

Vinylaromatische Monomere, beispielsweise Styrol, α-Methylstyrol, Vinyltoluol oder Divinylbenzol werden üblicherweise durch katalytische Dehydrierung entsprechender Alkylaromaten gewonnen. Neben dem Wertprodukt enthält das Reaktionsgemisch noch das Ausgangsprodukt, verschiedene vinyl- und alkylaromatische Nebenprodukte, sowie Oligomere des gewünschten Monomeren.

Die übliche Methode zur Gewinnung des gewünschten Monomeren in reiner Form ist die fraktionierte Destillation, bei der niedrigsiedende Bestandteile des Gemisches zunächst abgetrennt werden und schließlich das gewünschte Monomere von hochsiedenden Verbindungen abgetrennt wird.

Diese destillativen Trennungen werden dadurch kompliziert, daß Monomere mit steigender Temperatur zur Polymerisation neigen. Die Folge sind Ausbeuteverminderung und möglicherweise Polymeranwachsungen in der Apparatur, die zur Abstellung der Anlage führen können.

Bekanntlich kann die Bildung unerwünschten Polymerisats durch zwei Maßnahmen gemindert werden:

— durch Temperaturabsenkung durch Vakuumdestillation und
— durch Zugabe von Polymerisationsinhibitoren, beispielsweise Schwefel, tert.-Butylkatechol, Hydrochinon, Nitrosoverbindungen und dergleichen, vgl. JP-A 56 086 124, GB-A 2 056 481 und US-A 3 988 212.

Je nach Flüchtigkeit des Polymerisationsinhibitors unterscheidet man zwischen solchen, die vorzugsweise in der Gasphase und solchen, die in der Flüssigphase wirken, vgl. US-A 4 396 462 und US-A 4 442 048.

In der DE-B 1 040 020 ist ein Verfahren zur Verhinderung der Bildung von Sproßpolymerisat beim Destillieren von Vinyltoluol und Divinylbenzol enthaltender Gemische, die aus der thermischen Crackung stammen, beschrieben. Die Destillation wird dabei in Gegenwart von Nitroverbindungen als Inhibitor durchgeführt, wobei (vgl. Beispiel 1, Spalte 4, Zeilen 40–45) in einer mit Keramikfüllkörpern gepackten Kolonne gearbeitet wird. Die gesamte Menge des Inhibitors wird (vgl. Spalte 3, Zeilen 28–34 in Verbindung mit der Abbildung) durch Leitung 13 zusammen mit Kondensat über Leitung 14 in den oberen Abschnitt der Füllkörperkolonne zugeführt und zwar in einer Menge von 0,01 bis 0,1 Gew.-%, bezogen auf vinylaromatisches Ausgangsmaterial (vgl. Anspruch 2).

In den letzten Jahren werden die für die Vakuumrektifikation üblicherweise eingesetzten Füllkörper- und Bodenkolonnen in zunehmendem Maße durch Packungskolonnen verdrängt. Diese zeichnen sich durch höhere Trennleistung pro Stufe und geringeren Druckverlust aus.

Besonders geeignet sind solche druckverlustarmen Packungen, wenn zur Energieeinsparung mit Brüdenkompression gearbeitet wird.

Speziell bei der Trennung von Ethylbenzol/Styrol-Gemisch nutzt man den geringen Druckverlust solcher Kolonnen, jedoch mit unterschiedlicher Zielsetzung. Die folgenden drei Fälle kommen hier in Betracht:

1. Absenkung der Sumpftemperatur und Reduzierung der Verluste durch verminderte Polymerisatbildung;
2. Steigerung der Destillationskapazität, wobei im Extremfall Sumpftemperaturen wie bei entsprechenden Bodenkolonnen eingestellt werden; und
3. Betrieb der Packungskolonne mit Brüdenverdichtung. Dabei ist eine möglichst hohe Brüdendichte am Kolonnenkopf erforderlich, will man die geometrischen Abmessungen des Brüdenkompressors in vernünftigen Grenzen halten.

In den Fällen 2 und 3 treten nun dadurch Schwierigkeiten auf, daß die Polymerisatbildung im Vergleich zu Bodenkolonnen nicht — wie im obigen Fall 1 — abnimmt, sondern extrem zunimmt.

Die Verhältnisse im Abtriebsteil, d.h. dem unteren Teil der Kolonne, sind mit denen in Bodenkolonnen vergleichbar. Durch Zugabe geeigneter Polymerisationsinhibitoren, beispielsweise in den Kolonnenzulauf, läßt sich die Polymerisatbildung weitestgehend unterdrücken.

Ganz anders sind jedoch die Verhältnisse im oberen Teil der Kolonne, im sogenannten Verstärkungsteil. Durch den für eine Packungskolonne charakteristischen geringen Druckverlust ergeben sich hier deutlich höhere Drucke und Temperaturen, die, wenngleich für die Brüdenkompression durchaus erwünscht, die gegenüber Bodenkolonnen drastisch erhöhte Polymerisatbildung im inhibitorfreien Verstärkungsteil bewirken.

Die sich durch die starke Polymerisatbildung ergebenden tatsächlichen Ausbeuteverluste an Styrol sind sogar noch höher als der gebildete Polymeranteil. Bei der Rückstandsaufarbeitung, die beispielsweise im Dünnschichtverdampfer (vgl. US-A 3 515 647) erfolgt, verbleiben nämlich umso höhere Styrolanteile im Rückstand, je höher polymerhaltig derselbe ist.

Der Erfindung liegt die Aufgabe zugrunde, das eingangs angegebene Verfahren so zu modifizieren, daß die Polymerisatbildung auf einfache Weise unterdrückt oder doch verringert werden kann.

Diese Aufgabe wird dadurch gelöst, daß man die Destillation in einer Packungskolonne mit Packungssektionen in geregelter Anordnung durchgeführt und von dem Kolonnenzulauf, der ein Gemisch darstellt, welches Styrol und den Polymerisationsinhibitor enthält, einen Teilstrom von 1 bis 7 Gew.-%, bezogen auf den Kolonnenzulauf, abtrennt und diesen Teilstrom in den Flüssigkeitssammler unterhalb der obersten Packungssektion (Verstärkungsteil der Kolonne) einspeist und dabei eine Inhibitorkonzentration von 0,5 bis 20 ppm im Verstärkerteil aufrechterhält.

Mit dem erfindungsgemäßen Verfahren läßt sich nicht nur die Polymerisatbildung auf ein Minimum reduzieren, man kann auch Polymerisationsinhibitoren mit geringem Dampfdruck einsetzen, so daß eine Verunreinigung des Kopfproduktes mit dem Inhibitor vermieden wird.

Beim erfindungsgemäßen Verfahren wird gewöhnlich ein Gemisch aus Styrol und Ethylbenzol in vergleichbaren Mengen eingesetzt, wie das nachstehende Beispiel zeigt.

Der Polymerisationsinhibitor ist bereits im Kolonnenzulauf enthalten, und zwar vorteilhaft in einer Menge von 100 bis 2000 ppm, bezogen auf monomeres Styrol; besonders bevorzugt ist eine Menge von 200 bis 1000 ppm.

Der Teilstrom wird in einer Menge von 1 bis 7 Gew.-% des Kolonnenzulaufs, vorzugsweise 2 bis 6 Gew.-% des Zulaufs, eingesetzt. In diesem Fall beträgt die Inhibitorkonzentration im Verstärkungsteil der Kolonne vorzugsweise 0,5 bis 200 ppm, insbesondere 1 bis 10 ppm, bezogen auf die Rücklaufmenge.

Vorzugsweise wird im Kolonnenkopf eine Temperatur von 70 bis 120°C aufrecht erhalten. Die Temperatur im Kolonnensumpf beträgt vorzugsweise 90 bis 130°C.

Als geeignete Inhibitoren, Inhibitorengemische und Inhibitorenzubereitungen kommen eine ganze Reihe bekannter Agenzien in Betracht. So kann man p-Nitrosophenol gemäß GB-A 2 056 481, oder ein Gemisch aus p-Nitrosophenol und 2-sec.Butyl-4,6-dinitrophenol einsetzen. Man kann 2,6-Dinitro-p-cresol/Phenylendiamin oder 4-tert.Butylcatechol in Gegenwart von Sauerstoff gemäß der US-A 4 468 343 einsetzen. Es ist aber auch möglich, gemäß der US-A 4 466 904 mit Phenothiazin/4-tert. Butylcatechol oder 2,6-Dinitro-p-cresol in Gegenwart von Sauerstoff zu arbeiten. Wie in der US-A 3 988 212 vorgeschlagen wird, läßt sich auch ein Gemisch von N-Nitroso-diphenylamin/Dinitro-o-cresol einsetzen. Ebenso ist eine Kombination aus 2,6-Dinitro-4-ethylphenol/2,6-Dinitro-p-cresol gemäß US-A 4 457 806 möglich. Schließlich lassen sich auch Gemische, welche 2,6-Dinitro-4-halophenol enthalten, einsetzen, so wie das in der US-A 4 474 646 vorgeschlagen wird.

Besonders bevorzugt ist eine Kombination von p-Nitrosophenol und 2-sec.-Butyl-4,6-dinitrophenol. Besonders günstig ist bei der zuletzt genannten Kombination ein ungefähres Verhältnis von 400 ppm p-Nitrosophenol zu 150 ppm 2-sec.Butyl-4,6-dinitrophenol.

In den Zeichnungen bedeuten:

Figur 1 eine Siebbodenkolonne nach dem Stand der Technik, welche zwar nur geringe Mengen an unerwünschtem Polymerisat liefert, die jedoch hinsichtlich Durchsatz zu wünschen übrig läßt (Vergleichsbeispiel 1);

Figur 2 eine übliche Packungskolonne, bei der als Kolonnenzulauf ein Gemisch aus vinylaromatischer Verbindung, alkylaromatischer Verbindung und Polymerisationsinhibitor eingespeist wird (Vergleichsbeispiel 2); und

Figur 3 eine Packungskolonne, so wie sie in Figur 2 dargestellt ist, bei der erfindungsgemäß ein Teilstrom des einzuspeisenden Gemischs unterhalb des Kolonnenkopfes eingeführt wird (Beispiel).

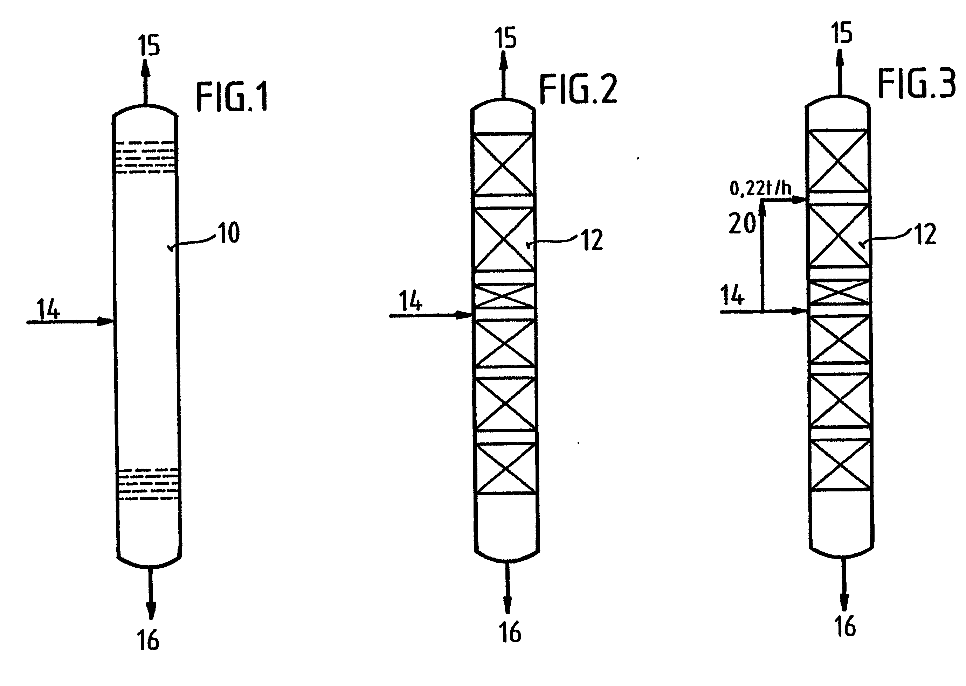

Die Erfindung wird unter Bezugnahme auf die Figuren 1 bis 3 in den nachstehenden Vergleichsbeispielen 1 und 2 und dem Beispiel näher erläutert.

Vergleichsbeispiel 1

Zur Trennung eines Ethylbenzol/Styrol(monomer)-Gemisches wird eine Siebbodenkolonne (10) mit 70 Böden (UCC) verwendet. Der Zulauf hat die ungefähre Zusammensetzung von 45 Gew.-% Styrol, 53 Gew.-% Ethylbenzol, 0,7 bis 0,8 Gew.-% Benzol, 2,0 bis 2,5 Gew.-% Toluol und 0,3 Gew.-% Rückstand. Der Zulauf enthält die folgenden Inhibitoren:

400 ppm p-Nitrosophenol
150 ppm 2-sec.Butyl-4,6-dinitrophenol (DNBP)
(jeweils bezogen auf Styrol).

Zulaufmenge (14) und Rücklaufverhältnis werden so gewählt, daß sich in der angegebenen Destillationseinrichtung folgende Werte ergeben:

Sumpftemperatur: 115°C
Destillat (15): 2% Styrol
Rohstyrol (16): 0,1% Ethylbenzol

Die übrigen Betriebsdaten stellen sich wie folgt ein:

Kopfdruck 71 mbar
Sumpfdruck 414 mbar
Druckverlust 343 mbar
Kopftemperatur 66°C
Zulaufmenge (14) 3,35 t/h
Polymergehalt im Rohstyrol (16) 0,06%

Vergleichsbeispiel 2

Als Destillationseinrichtung dient nunmehr eine Kolonne (12), die mit einer druckverlustarmen Packung in geregelter Anordnung (Sulzer-Mellapak) ausgestattet ist. Ihre äußeren Abmessungen sind denen der Siebbodenkolonne (10) aus Vergleichsbeispiel 1 ähnlich. Zulaufzusammensetzung, Inhibitorart und -konzentration entsprechen denen in Vergleichsbeispiel 1.

Wieder werden Zulaufmenge (14) und Rücklaufverhältnis so gewählt, daß die Werte für
Sumpftemperatur,
Styrolgehalt im Destillat (15),
Ethylbenzol-Gehalt im Rohstyrol (16),
denen aus Vergleichsbeispiel 1 entsprechen.

Dabei ergeben sich folgende weitere Betriebsdaten:

Kopfdruck 347 mbar
Sumpfdruck 414 mbar
Druckverlust 67 mbar Kopftemperatur 100°C
Zulaufmenge (14) 5,53 t/h
Polymergehalt im Rohstyrol (16) 0,40%.

Beispiel (erfindungsgemäß)

Die Destillationseinrichtung ist identisch mit der vom Vergleichsbeispiel 2. Zur Stabilisierung des Verstärkungsteils werden jedoch 4 Gew.-% der Zulaufmenge (14), d. s. 0,22 t/h, abgezweigt (20) und in den Flüssigkeitssammler unterhalb der obersten Packungssektion der Kolonne eindosiert. Bezogen auf die Rücklaufmenge ergibt sich eine Inhibitorkonzentration im Verstärkungsteil von
ca. 2 ppm p-Nitrosophenol und
ca. 1 ppm DNBP.
Sonst sind alle Verfahrensparameter identisch mit Vergleichsbeispiel 2. Der Polymergehalt im Rohstyrol beträgt nun 0,05%.
Es ist somit zu ersehen, daß bei Anwendung der erfindungsgemäßen Maßnahmen der Polymergehalt im Rohstyrol von 0,40 auf 0,05% absinken. Das entspricht einer Abnahme an Polymeren von 87,5%.

**Patentansprüche**

1. Verfahren zur Destillation von Styrol unter Vermeidung unerwünschter Polymerisation in einer Destillationskolonne mit Verstärkungsteil, wobei als Kolonnenzulauf ein Gemisch mit einem Gehalt an Styrol und Ethylbenzol in die Kolonne eingespeist wird und wobei ferner mindestens ein Polymerisationsinhibitor verwendet wird, dadurch gekennzeichnet, daß man die Destillation in einer Packungskolonne mit Packungssektionen in geregelter Anordnung durchführt und von dem Kolonnenzulauf, der ein Gemisch darstellt, welches Styrol und den Polymerisationsinhibitor enthält, einen Teilstrom von 1 bis 7 Gew.-%, bezogen auf den Kolonnenzulauf, abtrennt und diesen Teilstrom in den Flüssigkeitssammler unterhalb der obersten Packungssektion (Verstärkungsteil der Kolonne) einspeist und dabei eine Inhibitorkonzentration von 0,5 bis 20 ppm im Verstärkerteil aufrechterhält.

2. Verfahren nach Anspruch 1, dadurch gekennzeichnet, daß man im Kolonnenkopf eine Temperatur von 70 bis 120°C und im Kolonnensumpf eine Temperatur von 90 bis 130°C aufrechterhält.

**Claims**

1. A process for the distillation of styrene, which avoids undesirable polymerization, in a distillation column having a rectifying section, the column feed being a mixture containing styrene and ethylbenzene, and one or more polymerization inhibitors also being used, wherein the distillation is carried out in a column containing stacked packing sections and a bleed stream of from 1 to 7% by weight, based on the column feed, is separated off from the column feed, which is a mixture containing styrene and the polymerization inhibitor, and this bleed stream is fed into the liquid collector below the uppermost packing section (rectifying section of the column) and an inhibitor concentration of from 0.5 to 20 ppm is maintained in the rectifying section.

2. A process as claimed in claim 1, wherein the top of the column is maintained at from 70 to 120°C and the bottom of the column at from 90 to 130°C.

**Revendications**

1. Procédé de distillation de styrène en évitant une polymérisation indésirable dans une colonne de distillation à section d'enrichissement, un mélange à teneur en styrène, et éthylbenzène étant introduit dans la colonne, en tant qu'alimentation de colonne, et, en outre, au moins un inhibiteur de polymérisation étant utilisé, caractérisé par le fait que l'on effectue la distillation dans une colonne à remplissage, à sections de corps de remplissage en disposition réglée et, de l'alimentation de colonne, qui représente un mélange contenant du styrène et l'inhibiteur de polymérisation, on sépare un courant partiel de 1 à 7% en poids, rapportés à l'alimentation de colonne, et on introduit ce courant partiel dans le collecteur de liquide en-dessous de la section supérieure des corps de remplissage (section d'enrichissement de la colonne), et on y maintient une concentration d'inhibiteur de 0,5 à 20 ppm dans la section d'enrichissement.

2. Procédé selon la revendication 1, caractérisé par le fait que l'on maintient en tête de colonne, une température de 70 à 120°C et, en bas de colonne, une température de 90 à 130°C.

FIG.1

FIG.2

FIG.3